(19) Europäisches Patentamt
European Patent Office
Office européen des brevets

(11) Publication number: **0 223 126 B1**

## (12) EUROPEAN PATENT SPECIFICATION

(45) Date of publication of patent specification: **09.10.91**    (51) Int. Cl.5: **A61M 1/34**

(21) Application number: **86115019.1**

(22) Date of filing: **29.10.86**

(54) **A system for the collection, treatment and return of a patient's blood.**

(30) Priority: **18.11.85 SE 8505438**

(43) Date of publication of application:
**27.05.87 Bulletin 87/22**

(45) Publication of the grant of the patent:
**09.10.91 Bulletin 91/41**

(84) Designated Contracting States:
**BE CH DE ES FR GB IT LI NL**

(56) References cited:
| | |
|---|---|
| **EP-A- 0 122 748** | **EP-A- 0 171 749** |
| **US-A- 3 965 896** | **US-A- 4 191 182** |
| **US-A- 4 498 983** | **US-A- 4 547 186** |

(73) Proprietor: **GAMBRO AB**
**Post Box 10101**
**S-220 10 Lund(SE)**

(72) Inventor: **Solem, Jan Otto**
**Nordmannavägen 20**
**S-237 00 Bjärred(SE)**
Inventor: **Pilman, Olev Hugo**
**Karl XI:s gata 1**
**S-222 20 Lund(SE)**

(74) Representative: **Boberg, Nils Gunnar Erik**
**Gambro AB Patent Department Box 10101**
**S-220 10 Lund(SE)**

## Description

### TECHNICAL FIELD

The present invention relates to an autotransfusion system for the collection, treatment and return of a patient's blood, comprising means for the performance of the respective steps, including a suction device suitable for suction of blood from an open wound, means for adding a treatment liquid to the blood and separate means for returning the treated blood to the patient.

The system is intended primarily to be used postoperatively, e g in intensive-care wards after major operations. It is also suitable, though, for direct use during operations where major haemorrhages may occur, e g in connection with vascular surgery, liver operations and orthopaedic operations, such as hip-joint operations.

### BACKGROUND ART

Available systems for similar purposes, which are marketed, for example, under the name of Haemonetics Cell Saver, IBM Cell Washer and Dideco, all work with centrifuging and, after a careful wash, give a final product containing only red blood cells in a salt solution. These systems are also relatively expensive, owing to the centrifuges included. As an example of a system of this type, reference is made to the article "Autotransfusion and emergency surgery; preliminary report on an improved technique" in The International Journal of Artificil Organs, vol 8, no 4, 1985, p 221-224.

Simpler systems also exist which are sold, for example, under the name of Sorenson and Solcotrans. These systems consist quite simply of a plastic canister which is prefilled with a certain amount of ACD-solution and is proivded with a built-in filter. When the canister has been filled with blood it is quite simply tuned upside down, so that the blood can be retransfused through the filter. These simpler systems, however, are not sufficiently effective in the case of greater haemorrhages and, moreover, the returned blood frequently also contains amounts of washing liquids, anticoagulants and other additivs.

Reference is also being made to US-A-3 965 896 and US-A-4 547 186, describing two different autotransfusion systems including filters. Said filters are, however, no fluid separating filters and remove therefore only particles and the like.

### BACKGROUND INVENTION

It is an object of the invention to provide a simple system of the type defined above with the help of which it is possible to return wholly or partly cleaned whole blood, including its plasma and many other valuable substances, such as coagulation factors and the like.

The whole blood, moreover, will be concentrated to a suitable haematocrit value in that superfluous salt solution and anticoagulant solution are filtered off.

More particularly the system in accordance with the invention is characterized in that the said means for treatment of the blood comprises a fluid separating filter with a fluid inlet, and two fluid outlets, with the help of which superfluous fluid, including a part of said treatment liquid, can be removed through one of said outlets so as to concentrate and clean the blood intended to be returned to the patient.

The filter is preferably included in a recirculation circuit which preferably also comprises a peristaltic pump for the recirculation of the blood. Thus it becomes possible to select a filter which has a limited capacity and which, therefore, can be relatively inexpensive, so that it can be included in a treatment set intended for single use. The use of a peristaltic pump also facilitates the setting up of such a treatment set for single use, since the pump can be adapted to work on a conventional pump segment included in this treatment set. The effective parts of the pump thus do not have to come into direct contact with the blood.

The recirculation circuit appropriately comprises, moreover, a collecting vessel for treated blood or blood under treatment. This make it possible to work with continuous treatment of the blood and possibly also with continuous return of the blood, even if the same is supplied intermittently to the system. If this collecting vessel is provided with means for suspending it, the blood can be returned to the patient solely by means of gravity.

If the filtrate side of the filter is connected to a flexible fitrate bag for the collection of the filtrate without contact with the surrounding atmosphere, the risk of contamination is diminished. Owing to this it is possible to use an expendable filter for a longer period than if it were connected to an open drainage.

Preferably the said means for collecting the blood is constituted of a suction device with double ducts, one of which is adapted so as to be used for drawing up the blood, whereas the other is adapted for the supply of anticoagulant, e g an ACD-solution (Acid-Citrate-Dextrose), any excess of which is arranged to be removed with the help of the filter. Postoperatively in particular, however, it may be connected to a catheter for the direct drawing off of blood from the site of the operation.

The said means for treatment of the blood may comprise means for the supply of diluting fluid which may be constituted of a physiological salt solution which protects especially the red blood corpuscles and which can be removed with the help of the filter prior to the return of the blood to the patient. At the same time a certain amount of washing takes place with the help of this diluting fluid.

It should be noted that even without any supply of diluting fluid, the blood may have to be concentrated prior to return in order to remove, for example, an excess of anticoagulant or rinse fluid conveyed to the site of the operation.

Especially in the event of minor haemorrhages it may be suitable if the said means for the collecting of blood comprises an intermediate storage vessel. This may be constituted, for example, of a conventional cardiotomy reservoir provided with filter for filtering the blood. In this way the treatment of the blood may be deferred until the amount of blood collected is such as to make it worth while to employ an expendable set for the continued treatment.

If an intermediate storage vessel is used the said suction as well as the said means for the supply of diluting fluid may be connected to this, so that the dilution of the blood takes place in the same.

Particularly in postoperative use it may be appropriate instead to prefill this intermediate storage vessel with diluent and/or anticoagulant.

The system in accordance with the invention is provided appropriately with means for the control of the flow of anticoagulant, diluent and/or retransfusion blood. These means may be constituted, for example, of simple drip chambers in the respective ducts.

Preferably means are provided for the coupling together and/or taking apart of the system after the intermediate storage vessel, but before the filter and also before any recirculation circuit. In this way the system can be divided into two expendable sets, one comprising the intermediate storage vessel and parts arranged upstream of this in the system, and one comprising parts downstream of the intermediate storage vessel that is to say especially the filter and other parts for the concentration of the blood.

## BRIEF DESCRIPTION OF DRAWING

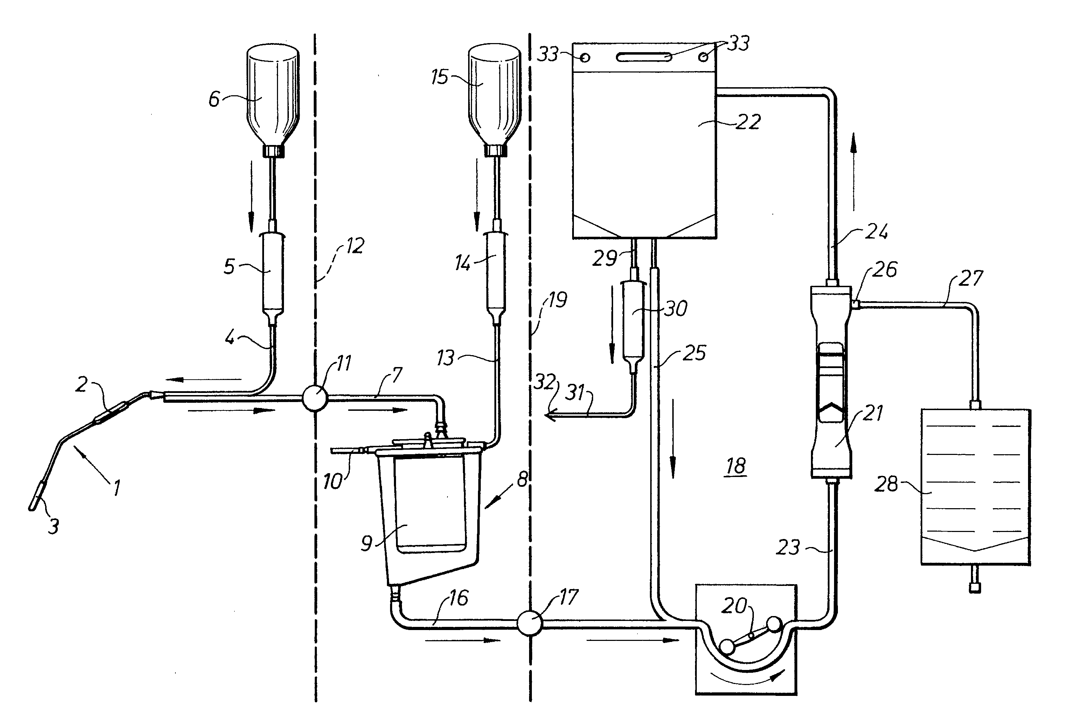

The invention will be described in more detail in the following with reference to the attached drawing which, by the way of example, shows schematically a preferred embodiment of the same.

## BEST MODE OF CARRYING OUT THE INVENTION

The preferred embodiment of a system in accordance with the invention shown on the attached drawing by way of example comprises a suction device 1 with a handling device 2 and a suction tip 3 which appropriately should be designed as a filter basket in order to prevent any drawing up of lumps or the like. The suction device 1 is provided, in a manner not shown, with two ducts, one of which is adapted to be used for the supply of an anticoagulant which is supplied via a duct 4 with a monitoring device 5 from a source 6, e g a bottle containing ACD. The blood drawn up by means of the suction device 1 is led through a duct 7 to an intermediate storage vessel 8 which in the present embodiment is constituted of a conventional cardiotomy reservior provided with a filter 9 for filtering the blood. The cardiotomy reservoir 8 is connected via a duct 10 to a source of vacuum, not shown in the drawing. At 11 is indicated how the system in accordance with the invention with the help, for example, of a coupling can be divided into a suction part on the left of the broken line 12 and a treatment part on the right of the same line. The firstname part is not required, therefore, if the duct 7, for example, in postoperative application, is connected directly to a draw-off catheter connected to the site of the operation.

To the cardiotomy reservoir 8 is connected, moreover, a duct 13 for the conveying of diluting fluid, e g a physiological salt solution, via a monitoring device 14 from a source 15 for such a solution. This solution is then mixed with the blood in the reservoir 8. From this reservoir the mixture is conducted with the help of a duct 16 via a further coupling 17, shown schematically, to a recirculation circuit which in its entirety is designated by 18. The system also can be divided, therefore, with the help of the coupling 17 along the broken line 19, the parts to the left of this line being intended primarily for the collection and dilution of the blood, wheras the parts to the right of the same are intended for the concentration and return of the blood to the patient. In the recirculation circuit 18 the blood is pumped with the help of a peristaltic pump 20 through a filter 21 to a collecting vessel 22. This is done with the help of the ducts 23 and 24 and a shunt duct 25. The outlet 26 for the filtrate is connected via a duct 27 to a flexible filtrate bag 28 for the collection of the filtrate without contact with the surrounding atmosphere. When the treatment of the blood is finished, that is to say when the appropriate haematocrit value has been reached, it is passed via a duct 29 with a monitoring device 30 to a retransfusion duct 31, whose tip 32 is intended to symbolize an injection cannula. The collecting vessel 22 is provided appropriately with means for its suspension. These means have been designated in the figure by 33 and consist in the example shown of a handle and two suspension eyes.

The filter 21 is constituted appropriately of a filter of the type used normally for haemofiltration. If a more rapid concentration is desired, e g in connection with a continuous treatment of a patient where there is a risk of heavy bleeding, it is possible instead to use a filter of the type used normally for plasmapheresis. Examples of membrane material intended for haemofiltration are found, for example, in EP 0 046 816 and EP 0 098 392. In the same way examples of membranes suitable for plasmapheresis are found in EP 0 044 958 and EP 0 095 554.

Naturally the invention is not limited simply to the embodiment described above, but may be varied within the scope of the following claims. For example the parts included in the system may be varied within wide limits with regard to their form as well as their function.

## Claims

1. An autotransfusion system for the collection, treatment and return of a patient's blood, comprising means for the performance of the respective steps, including, a suction device (1) suitable for suction of blood from an open wound, means (4,5,6 and/or 15,14,13) for adding a treatment liquid to the blood and separate means (31,32) for returning the treated blood to the patient, **characterized** in that said means for the treatment of the blood comprises a fluid separating filter (21) with a fluid inlet, and two fluid outlets, with the help of which superfluous fluid, including a part of said treatment liquid, can be removed through one of said outlets (26) so as to concentrate and clean the blood intended to be returned to the patient.

2. A system in accordance with claim 1, **characterized** in that the said filter is included in a recirculation circuit (18) which preferably also comprises a peristaltic pump (20) for the recirculation of the blood.

3. A system in accordance with claim 2, **characterized** in that the recirculation circuit (18) comprises, moreover, a collecting vessel (22) for treated blood or blood under treatment.

4. A system in accordance with anyone of the preceding claims, **characterized** in that the filtrate side (26) of the filter (21) is connected to a flexible filtrate bag (28) for the collection of the filtrate without contact with the surrounding atmosphere.

5. A system in accordance with anyone of the preceding claims, **characterized** in that the said means for collecting the blood is constituted of a suction device (1) with double ducts, one of which is adapted so as to be used for drawing up the blood, whereas the other is adapted for the supply of anticoagulant, any excess of which is arranged to be removed with the help of the filter.

6. A system in accordance with anyone of the preceding claims, **characterized** in that the said means for the treatment of the blood comprises means (13,14,15) for the supply of diluting fluid.

7. A system in accordance with anyone of the preceding claims, **characterized** in that the said means for collecting the blood comprises an intermediate storage vessel (8) which preferably is constituted of a conventional cardiotomy reservoir provided with a filter for the filtering of the blood.

8. A system in accordance with claims 5-7, **characterized** in that the said suction device and the said means (13,14,15) for the supply of diluting fluid are connected to the intermediate storage container (8), so that the diluting of the blood takes place in the same.

9. A system in accordance with anyone of claims 5-8, **characterized** by means (5,14,30) for the control of flow of anticoagulant, diluent and/or the retransfusion of blood.

10. A system in accordance with claim 3, **characterized** by means (33) for the suspension of the said collecting vessel for treated blood, so that the latter can be returned to the patient solely by means of gravity.

11. A system in accordance with anyone of claims 7-9, **characterized** by means (17) for the coupling together and/or taking apart of the system arranged after the intermediate storage vessel (8) but before the filter (21) and also before any recirculation circuit (18).

## Revendications

1. Système d'autotransfusion pour la collecte, le traitement et la restitution du sang d'un malade, comprenant un moyen pour réaliser les étapes respectives, incluant un dispositif de succion (1) approprié pour la succion du sang à partir d'une blessure ouverte, des moyens (4, 5, 6 et/ou 15, 14, 13) pour ajouter un liquide de traitement au sang et des moyens séparés (31, 32) pour restituer le sang traité au malade, caractérisé en ce que ledit moyen pour le traitement du sang comprend un filtre séparateur de fluide (21) avec une entrée de fluide, et deux sorties de fluide, à l'aide desquels on peut enlever le fluide superflu, y compris une partie dudit liquide de traitement, à travers une desdites sorties (26), de manière à concentrer et à purifier le sang devant être restitué au malade.

2. Système selon la revendication 1, caractérisé en ce que ledit filtre est inclus dans un circuit de recyclage (18) qui de préférence comprend également une pompe péristaltique (20) pour le recyclage du sang.

3. Système selon la revendication 2, caractérisé en ce que le circuit de recyclage (18) comprend de plus un récipient de collecte (22) pour le sang traité ou le sang en cours de traitement.

4. Système selon l'une quelconque des revendications précédentes, caractérisé en ce que le côté filtrat (26) du filtre (21) est relié à un sac de filtrat flexible (28) pour la collecte du filtrat sans contact avec l'atmosphère environnante.

5. Système selon l'une quelconque des revendications précédentes, caractérisé en ce que ledit moyen pour collecter le sang est constitué d'un dispositif de succion (1) avec doubles conduits, dont l'un est adapté de manière à être utilisé pour tirer le sang, tandis que l'autre est adapté pour la fourniture d'un anti-coagulant, dont tout excès peut être retiré à l'aide du filtre.

6. Système selon l'une quelconque des revendications précédentes, caractérisé en ce que ledit moyen pour le traitement du sang comprend des moyens (13, 14, 15) pour la fourniture du fluide diluant.

7. Système selon l'une quelconque des revendications précédentes, caractérisé en ce que ledit moyen pour collecter le sang comprend un récipient de stockage intermédiaire (8) qui de préférence est constitué d'un réservoir de cardiotomie classique muni d'un filtre pour le filtrage du sang.

8. Système selon les revendications 5-7, caractérisé en ce que ledit dispositif de succion et lesdits moyens (13, 14, 15) pour la fourniture du fluide de dilution sont reliés au récipient de stockage intermédiaire (8), de manière que la dilution du sang s'y produise.

9. Système selon l'une quelconque des revendications 5-8, caractérisé par des moyens (5, 14, 30) pour la régulation du débit d'anticoagulant, de diluant et/ou la retransfusion du sang.

10. Système selon la revendication 3, caractérisé par des moyens (33) pour la suspension dudit récipient de collecte pour le sang traité, de manière que ce dernier puisse être restitué au malade uniquement au moyen de la gravité.

11. Système selon l'une quelconque des revendications 7-9, caractérisé par un moyen (17) pour le couplage et/ou la séparation du système disposé à travers le récipient de stockage intermédiaire (8) mais avant le filtre (21) et également avant tout circuit de recyclage (18).

**Patentansprüche**

1. Selbsttransfusionssystem für die Sammlung, die Behandlung und die Rückgabe des Blutes eines Patienten, mit einer Einrichtung für die Durchführung der entsprechenden Schritte einschließlich einer Saugeinrichtung (1), die für das Absaugen von Blut aus einer offenen Wunde geeignet ist, einer Einrichtung (4, 5, 6 und/oder 15, 14, 13) für die Zugabe einer Behandlungsflüssigkeit Zu dem Blut und mit einer getrennten Einrichtung (31, 32) für die Rückführung des behandelten Blutes zu dem Patienten, dadurch gekennzeichnet, daß die Einrichtung für die Blutbehandlung einen Fluidabtrennfilter (21) mit einem Fluideinlaß und zwei Fluidauslässen aufweist, mit dessen Hilfe ein überflüssiges Fluid, einschließlich eines Teils der Behandlungsflüssigkeit, durch einen der Auslässe (26) entfernt werden kann, um so das Blut, welches zu dem Patienten zurückgeführt werden soll, zu konzentrieren und zu reinigen.

2. System nach Anspruch 1, dadurch gekennzeichnet, daß der Filter in einem Rezirkulierungskreislauf (18) enthalten ist, der vorzugsweise auch eine peristaltische Pumpe (20) für die Rezirkulierung des Blutes aufweist.

3. System nach Anspruch 2, dadurch gekennzeichnet, daß der Rezirkulationskreislauf (18) darüberhinaus ein Sammelgefäß (22) für behandeltes Blut oder in der Behandlung befindliches Blut aufweist.

4. System nach einem der vorstehenden Ansprüche, dadurch gekennzeichnet, daß die Filtratseite (26) des Filters (21) mit einem flexiblen Filtratbeutel (28) verbunden ist, um das Filtrat ohne Kontakt mit der umgebenden Atmosphäre aufzusammeln.

5. System nach einem der vorstehenden Ansprüche, dadurch gekennzeichnet, daß die Einrichtung zum Sammeln des Blutes aus einer Saugeinrichtung (1) mit doppelten Leitungen besteht, von denen eine so ausgelegt ist, daß sie für das Abziehen des Blutes verwendet werden kann, während die andere für die Zufuhr eines Antikoagulans ausgelegt ist, wobei Vorkehrungen getroffen sind, daß jeglicher Überschuß desselben mit Hilfe des Filters entfernt wird.

6. System nach einem der vorstehenden Ansprüche, dadurch gekennzeichnet, daß die Einrichtung zur Blutbehandlung eine Einrichtung (13, 14, 15) für die Zufuhr eines Verdünnungsfluids aufweist.

7. System nach einem der vorstehenden Ansprüche, dadurch gekennzeichnet, daß die Einrichtung zum Sammeln des Blutes ein Zwischenspeichergefäß (8) aufweist, welches vorzugsweise aus einem konventionellen, Kardiotomie-Vorratsgefäß besteht, welches mit einem Filter für das Filtern des Blutes versehen ist.

8. System nach den Ansprüchen 5 bis 7, dadurch gekennzeichnet, daß die Saugeinrichtung und die Einrichtung (13, 14, 15) für die Zufuhr eines Verdünnungsfluids mit dem Zwischenspeicherbehälter (8) verbunden sind, so daß die Verdünnung des Blutes in diesem stattfindet.

9. System nach einem der Ansprüche 5 bis 8, gekennzeichnet durch eine Einrichtung (5, 14, 30) für die Steuerung des Stromes eines Antikoagulansmittels, eines Verdünnungsmittels und/oder des zurücktransfundierten Blutes.

10. System nach Anspruch 3, gekennzeichnet durch eine Einrichtung (33) für die Aufhängung des Sammelgefäßes für behandeltes Blut, so daß letzteres allein mit Hilfe der Schwerkraft zu dem Patienten zurückgeführt werden kann.

11. System nach einem der Ansprüche 7 bis 9, gekennzeichnet durch eine Einrichtung (17) für das Zusammenkoppeln und/oder Abtrennen des Systems, welches hinter dem Zwischenspeichergefäß (8), jedoch vor dem Filter (21) und auch vor irgendeinem Rezirkulationskreislauf (8) angeordnet ist.

EP 0 223 126 B1